# EUROPEAN PATENT APPLICATION

(11) **EP 2 181 640 A1**
(43) Date of publication of application: **05.05.2010**
(21) Application number: 08765035.4
(22) Date of filing: 03.06.2008
(51) Int. Cl.: A61B 1/00, A61B 5/06, A61B 5/07

(54) **INTRA-SPECIMEN POSITION DETECTING SYSTEM AND INTRA-SPECIMEN POSITION DETECTING METHOD**

(30) Priority: 28.08.2007 JP 2007221458
(71) Applicant: OLYMPUS CORPORATION, Shibuya-ku Tokyo 151-0072 (JP); OLYMPUS MEDICAL SYSTEMS CORP., Shibuya-ku Tokyo 151-0072 (JP)
(72) Inventor: TAMURA, Kazuaki, Tokyo 192-8512 (JP); MORI, Takeshi, Tokyo 192-8512 (JP); MINAI, Tetsuo, Tokyo 192-8512 (JP); UCHIYAMA, Akio, Tokyo 192-8512 (JP)
(74) Representative: Schicker, Silvia
(86) International application number: PCT/JP2008/060222
(87) International publication number: WO 2009/028246

(57) **Abstract**

From among plural receiver electrodes (6a to 6n) for receiving a transmission signal transmitted from transmitter electrodes (16a, 16b) of an intra-subject introduction device (2), receiver electrodes for receiving an image reception signal are selected by a receiver electrode selection circuit (18). Further, receiver electrodes for receiving a signal for position detection are selected by a receiver electrode selection circuit (21), from among the other ones of the plural receiver electrodes (6a to 6n) than the receiver electrodes selected by the receiver electrode selection circuit (18).

## Description

### Technical Field

The present invention relates to an intra-subject position detection system and an intra-subject position detection method capable of deriving positional information of an intra-subject introduction device introduced into a test subject.

### Background Art

Recently in the field of endoscopy, there has been a proposal for a capsule endoscope (intra-subject introduction device) of an orally administered type. The capsule endoscope has an imaging function and a wireless communication function. The capsule endoscope is swallowed by a test subject for the purpose of observation (inspection), and thereafter moves inside internal organs in a body cavity, such as a stomach and a small intestine, in accordance with peristaltic movement, to obtain intra-subject information such as image data until the endoscope is passed naturally. While the capsule moves inside the body cavity, image data which is obtained inside the subject by the capsule endoscope is sequentially transmitted to outside by wireless communications, and is stored in a memory provided externally.

As a wireless communication scheme for use with a capsule endoscope, there has been proposed a human body communication scheme which uses a test subject as a signal propagation medium to externally transmit image data. Relating to a capsule endoscope according to the human body communication scheme, there has been also proposed a scheme in which a function of performing position detection of a capsule endoscope is provided in the side of an external receiver in order to specify an imaging position of image data picked up by the capsule endoscope inside a test subject. As an example of an intra-subject position detection system equipped with such a position detection function, there has been proposed a system which uses, for position detection, a transmission signal transmitted from a capsule endoscope by utilizing a potential difference between electrodes provided on an outer circumferential surface of the capsule endoscope (for example, see Jpn. PCT National Publication No. 2006-513001). In the example of Jpn. PCT National Publication No. 2006-513001, a transmission signal transmitted from a capsule endoscope is received through a test subject by receiver electrodes of an external receiver provided outside the test subject. The position of the capsule endoscope is derived based on differences in reception intensity between the respective receiver electrodes. Further, according to a position detection method by using the external receiver disclosed in Jpn. PCT National Publication No. 2006-513001, receiver electrodes are sequentially selected from among plural receiver electrodes provided outside a test subject, and voltage intensities of signals obtained by the receiver electrodes are stored in a memory. Thereafter, optimal receiver electrodes for receiving signals are selected based on the voltage intensities stored in the memory.

### Disclosure of Invention

In case where position detection is performed while sequentially switching receiver electrodes after receiving image data, a time difference occurs between operation of receiving image data and position detection operation. If such a time difference occurs, an error can be incurred between position of a capsule endoscope at time of receiving image data and position of the capsule endoscope at time of position detection. If such an error occurs, the capsule endoscope lacks accuracy in position detection.

The present invention has been made in view of the situation as described above, and has as its object to provide an intra-subject position detection system capable of improving detection accuracy by performing position detection of a capsule endoscope at high speed.

An intra-subject position detection system of a first aspect of the invention, comprising: an intra-subject introduction device that is introduced into a test subject and obtains an intra-subject information, and a position detection device that is provided outside the test subject and derives a positional information of the intra-subject introduction device inside the test subject, **characterized in that** the intra-subject introduction device comprises transmitter electrodes that are positioned on an outer circumferential surface of the intra-subject introduction device, to transmit the intra-subject information to outside of the test subject, and the position detection device comprises a plurality of receiver electrodes provided on an outer surface of the test subject, a receiver electrode selection circuit that selects receiver electrodes for receiving the intra-subject information, from among the plurality of receiver electrodes, a position detection electrode selection circuit that selects receiver electrodes for position detection of the intra-subject introduction device, from among the other receiver electrodes of the plurality of receiver electrodes than the receiver electrodes selected by the receiver electrode selection circuit, and a positional information derivation circuit that derives the positional information of the intra-subject introduction device, based on an electric signal from the receiver electrodes selected by the position detection electrode selection circuit.

An intra-subject position detection method of a second aspect of the invention, for deriving a positional information of an intra-subject introduction device that is introduced into a test subject and obtains intra-subject information, the method comprising: selecting receiver electrodes for receiving the intra-subject information, from among a plurality of receiver electrodes provided on an outer surface of the test subject; selecting, from among the other receiver electrodes of the plurality of receiver electrodes than the selected receiver electrodes, receiver electrodes for a position detection of the intra-subject introduction device; and deriving the positional information of the intra-subject introduction device, based on an electric signal transmitted from transmitter electrodes and received by the receiver electrodes for the position detection, the transmitter electrodes being provided on an outer circumferential surface of the intra-subject introduction device, to transmit the intra-subject information to outside of the test subject.

### Brief Description of Drawings

FIG. 1 is a schematic view illustrating the whole configuration of an intra-subject position detection system according to the first embodiment of the invention;
FIG. 2 is a block diagram representing a detailed interior configuration of a capsule endoscope;
FIG. 3 is a block diagram representing a detailed configuration of interior of the position detection device according to the first embodiment;
FIG. 4A is a flowchart representing extraction operation for image data by a processing device;
FIG. 4B is a flowchart representing position detection operation by the processing device;
FIG. 5 is a chart representing operation timings of image extraction operation and position detection operation;
FIG. 6 is a plan view for describing a procedure of selecting receiver electrodes for position detection;
FIG. 7 is a block diagram representing a detailed interior configuration of a position detection device according to the second embodiment of the invention; and
FIG. 8 is a schematic view for describing position detection of a capsule endoscope by the position detection device according to the second embodiment of the invention, using together impedance between receiver electrodes.

### Best Mode for Carrying Out the Invention

Hereinafter, embodiments of the present invention will be described with reference to the drawings.

### [First Embodiment]

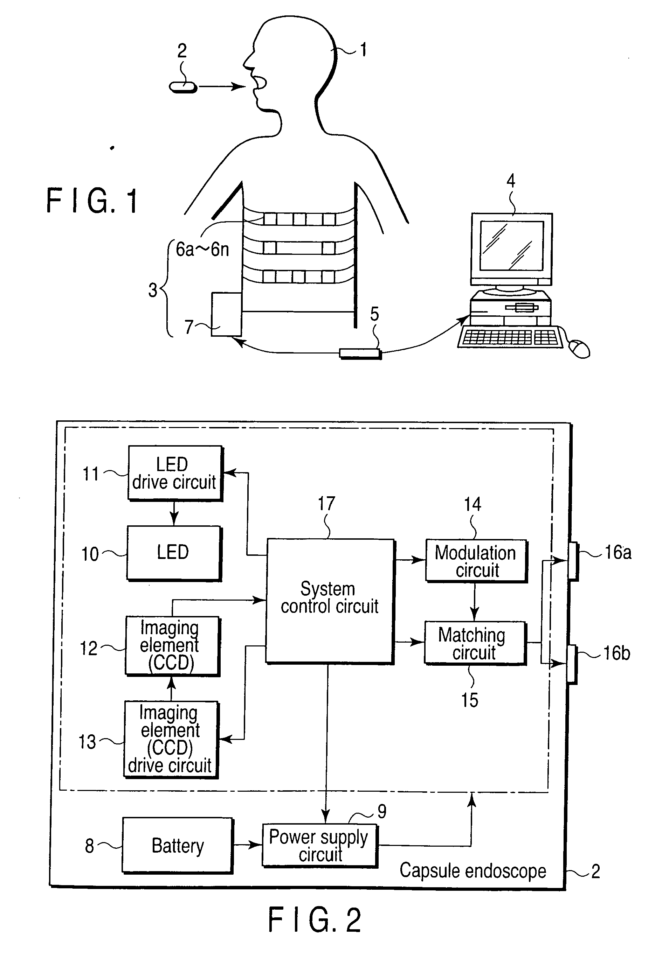

At first, the first embodiment of the invention will now be described. FIG. 1 is a schematic view illustrating the whole configuration of an intra-subject position detection system according to the first embodiment of the invention. A capsule endoscope system illustrated in FIG. 1 includes a capsule endoscope 2, a position detection device 3, a display device 4, and a mobile recording medium 5.

A capsule endoscope 2 which functions as an intra-subject introduction device is introduced into a test subject 1. The capsule endoscope 2 obtains intra-subject information (for example, image data of interior of the test subject 1) by repeatedly performing imaging while moving along a moving route inside the test subject 1.

The position detection device 3 makes communication with the capsule endoscope 2, and detects the position of the capsule endoscope 2. As illustrated in FIG. 1, the position detection device 3 includes receiver electrodes 6a to 6n and a processing device 7. The receiver electrodes 6a to 6n are provided on an outer surface of the test subject 1, to receive a transmission signal from the capsule endoscope 2. The processing device 7 derives an image of interior of the test subject 1 from the transmission signal received through the receiver electrodes 6a to 6n.

The display device 4 displays content of image data reproduced by the position detection device 3. The display device 4 is configured, for example, as a workstation which displays images based on data obtained by the mobile recording medium 5. More specifically, the display device 4 functions to reproduce video signals from data recorded on the mobile recording medium 5 and to display the reproduced video signals, for example, on a CRT display or a liquid crystal display.

The mobile recording medium 5 is attachable to and detachable from the processing device 7 and the display device 4. The mobile recording medium 5 is configured to be capable of outputting information to and recording information on the processing device 7 or the display device 4, when attached to the processing device 7 or the display device 4. Specifically, the mobile recording medium 5 is attached to the processing device 7 and records images of interior of the test subject and positional data of the capsule endoscope 2 while the capsule endoscope 2 is moving inside a body cavity in the test subject 1. After the capsule endoscope 2 is excreted from the test subject 1, the mobile recording medium 5 is taken out of the processing device 7 and attached to the display device 4. In this manner, data recorded on the mobile recording medium 5 is read by the display device 4.

FIG. 2 is a block diagram representing a detailed interior configuration of the capsule endoscope 2. The capsule endoscope 2 includes a battery 8, a power supply circuit 9, an LED 10, an LED drive circuit 11, an imaging element (CCD) 12, an imaging element (CCD) drive circuit 13, a modulation circuit 14, a matching circuit 15, transmitter electrodes 16a and 16b, and a system control circuit 17.

The battery 8 is a power supply which allows the capsule endoscope 2 to internally use electric power. The power supply circuit 9 generates electric power to be supplied for respective components inside the capsule endoscope 2 from the battery 8. The power supply circuit 9 supplies the electric power to respective components of the capsule endoscope 2. Respective components of the capsule endoscope 2 operate using, as drive energy, electric power supplied from the power supply circuit 9.

The LED 10 is a light source for illuminating an imaging area inside the test subject 1 when imaging interior of the test subject 1. The LED drive circuit 11 is a drive circuit for driving the LED 10. The imaging element 12 is an imaging element according to a CCD scheme, which obtains intra-subject information (image signals) by imaging at least a part of the imaging area illuminated by the LED 10. The imaging element drive circuit 13 is a drive circuit for driving the imaging element 12. Image signals obtained by the imaging element 12 are digitized by the system control circuit 17, thereby to generate image data of interior of the test subject 1.

Use of an LED and an imaging element according to the CCD scheme respectively as a light source and an imaging element is not mandatory. For example, an imaging element according to a CMOS scheme may be used instead.

The modulation circuit 14 performs processing, such as modulation, on image data of the test subject 1 which is obtained by the system control circuit 17, and thereby generates a transmission signal to transmit data to the position detection device 3.

The matching circuit 15 changes characteristic impedance of the transmission signal generated by the modulation circuit 14, in accordance with an instruction from the system control circuit 17, to perform impedance matching between the transmitter electrodes 16a and 16b and the test subject 1. Specifically, a variable impedance element inserted in series or parallel between the transmitter electrodes 16a and 16b is used as the matching circuit 15. Characteristics such as characteristic impedance, electric power, a phase, and a frequency of the transmission signal can be changed by the matching circuit 15 as described above. Further, the matching circuit 15 includes a current-protection resistance element for regulating a maximum value of a current flowing inside the test subject 1.

The transmitter electrodes 16a and 16b are electrodes for transmitting the transmission signal output from the matching circuit 15 to inside of the test subject 1. The transmitter electrodes 16a and 16b are made of metal which has electric conductivity and excellent resistance to corrosion and is harmless to human body. The transmitter electrodes 16a and 16b are provided on an outer circumferential surface of the capsule endoscope 2.

The system control circuit 17 controls operations of the LED drive circuit 11, imaging element drive circuit 13, modulation circuit 14, matching circuit 15, and power supply circuit 9, and generates image data of the test subject 1 from the image signal obtained by the imaging element 12.

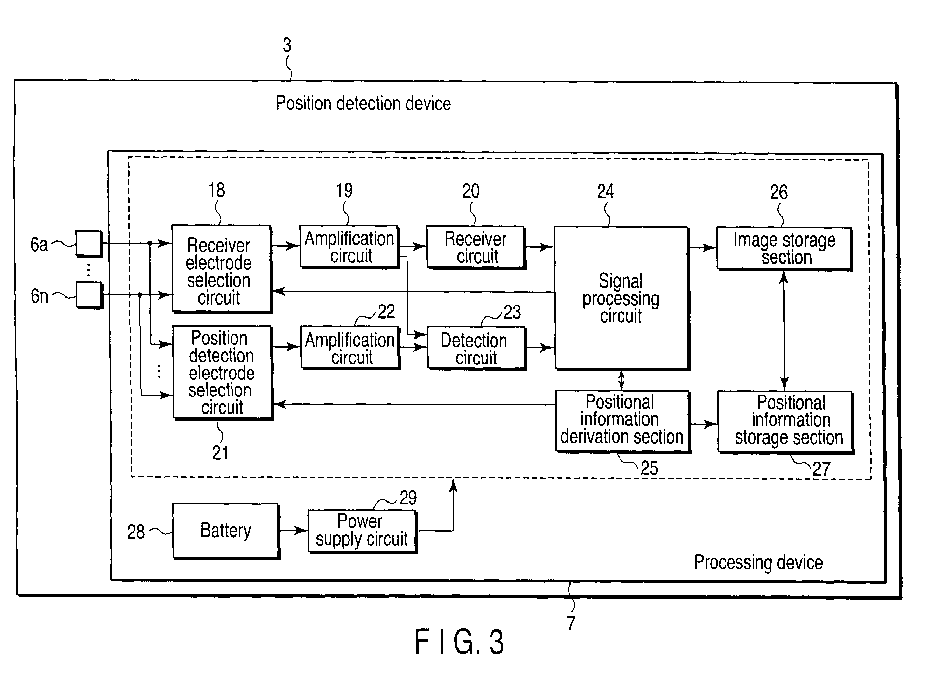

FIG. 3 is a block diagram representing a detailed configuration of interior of the position detection device 3 according to the first embodiment. As described above, the position detection device 3 includes the receiver electrodes 6a to 6n and the processing device 7.

The receiver electrodes 6a to 6n are electrodes for receiving a transmission signal transmitted from the transmitter electrodes 16a and 16b comprised in the capsule endoscope 2. Specifically, the receiver electrodes 6a to 6n are made of metal which has electric conductivity and excellent resistance to corrosion and is harmless to human body. The receiver electrodes 6a to 6n are located on a body surface of the capsule endoscope 2. The "n" is an integer not smaller than 3 and denotes a quantity of the receiver electrodes 6a to 6n. The transmission signal is received by using receiver electrodes which are selected by the processing device 7 from among the receiver electrodes 6a to 6n. The quantity of the receiver electrodes 6a to 6n, positions thereof to be located, and specific shapes thereof are not limited to those illustrated in FIG. 1 but may be configured arbitrarily. For example, FIG. 1 illustrates an example in which the receiver electrodes 6a to 6n are provided in front of the test subject 1. In actual, however, the receiver electrodes 6a to 6n may be provided even on sides and back of the test subject 1 so that three-dimensional position of the capsule endoscope 2 may be measured.

The processing device 7 has a function to receive the transmission signal transmitted from the capsule endoscope 2 and extract image data, and also a function to receive the transmission signal transmitted from the capsule endoscope 2 and detect the position and orientation of the capsule endoscope 2. The processing device 7 includes a receiver electrode selection circuit 18, an amplification circuit 19, a receiver circuit 20, a position detection electrode selection circuit 21, an amplification circuit 22, a detection circuit 23, a signal processing circuit 24, a positional information derivation section 25, an image storage section 26, a positional information storage section 27, a battery 28, and a power supply circuit 29.

The receiver electrode selection circuit 18 selects a set of receiver electrodes (hereinafter referred to as paired receiver electrodes) which are adequate for receiving the transmission signal, in accordance with a receiver electrode switching signal from the signal processing circuit 24. The amplification circuit 19 performs processing, such as differential amplification, on a reception signal (hereinafter referred to as an image reception signal) from the paired receiver electrodes selected by the receiver electrode selection circuit 18, and outputs an image reception signal subjected to the processing to the detection circuit 23 and the signal processing circuit 24. As an alternative, a band-pass filter (BPF) taking the frequency of the transmission signal of the capsule endoscope 2 as a center frequency may be mounted in the amplification circuit 19, to improve the signal-to-noise ratio of the image reception signals. If such a configuration is adopted, a low-pass filter (LPF) or a high-pass filter (HPF) may be used insofar as the low-pass or high-pass filter allows a frequency band of the transmission signal to pass. The receiver circuit 20 performs processing, such as demodulation, on the image reception signal amplified by the amplification circuit 19.

From among the receiver electrodes 6a to 6n, the position detection electrode selection circuit 21 selects, as electrodes for position detection, paired receiver electrodes other than the electrodes selected by the receiver electrode selection circuit 18, in accordance with a position detection electrode switching signal from the positional information derivation section 25. The amplification circuit 22 performs processing, such as differential amplification, on a position detection signal from the paired receiver electrodes selected by the position detection electrode selection circuit 21. In this respect, a band-pass filter (BPF) taking the frequency of the transmission signal of the capsule endoscope 2 as a center frequency may be mounted in the amplification circuit 22, to improve the signal-to-noise ratio of a position detection signal. The detection circuit 23 detects a signal intensity of a signal amplified by the amplification circuit 19 or 22. For example, a circuit which detects amplitude of a signal by smoothing a reception signal with use of a rectifier circuit and a smoothing circuit may be considered to be a specific configuration of the detection circuit 23. Alternatively, a signal intensity may be detected by using an effective value detection circuit (true RMS converter) which extracts an effective value of amplitude of a reception signal. Still alternatively, a signal intensity may be detected by using a peak detection circuit which detects a peak of a reception signal.

The signal processing circuit 24 extracts image data of interior of a test subject from an output signal of the receiver circuit 20. The signal processing circuit 24 selects paired receiver electrodes capable of most efficiently receiving a transmission signal (image reception signal) from the orientation and position of the capsule endoscope 2 which are derived by the positional information derivation section 25. The signal processing circuit 24 outputs a result thereof as a receiver electrode switching signal to the receiver electrode selection circuit 18.

The positional information derivation section 25 includes an unillustrated memory for storing signal intensities of reception signals which are received by the receiver electrodes selected by the receiver electrode selection circuits 18 and the position detection electrode selection circuit 21 and are input through the signal processing circuit 24 from the detection circuit 23. In the configuration as described above, the positional information derivation section 25 derives the orientation and position of the capsule endoscope 2 existing in a test subject, from the signal intensities of reception signals which are stored in the memory. The positional information derivation section 25 also outputs a position detection electrode switching signal to the position detection electrode selection circuit 21 so as to select receiver electrodes other than the receiver electrodes selected by the receiver electrode selection circuit 18.

The image storage section 26 stores image data extracted by the signal processing circuit 24. The positional information storage section 27 stores data the orientation and position of the capsule endoscope 2 derived by the positional information derivation section 25, with the data associated with image data which has been obtained at the same timing as the data of the orientation and position of the capsule endoscope 2 and stored in the image storage section 26. In this manner, the image data and an imaging position thereof can be associated with each other. Data stored in the image storage section 26 and positional information storage section 27 is written to the mobile recording medium 5 illustrated in FIG. 1 when the mobile recording medium 5 is attached to the processing device 7.

The battery 28 is a power supply for using electric power in the position detection device 3. The power supply circuit 29 generates electric power to be supplied to respective components in the position detection device 3 from the battery 28, and supplies the electric power to the respective components of the position detection device 3. The respective components of the position detection device 3 operate using, as drive energy, the electric power supplied from the power supply circuit 29.

Next, position detection operation of the intra-subject position detection system according to the first embodiment will be described. The position detection operation according to the present embodiment is closely related to extraction operation for image data. Therefore, the extraction operation for image data will be described along with the position detection operation in the following description.

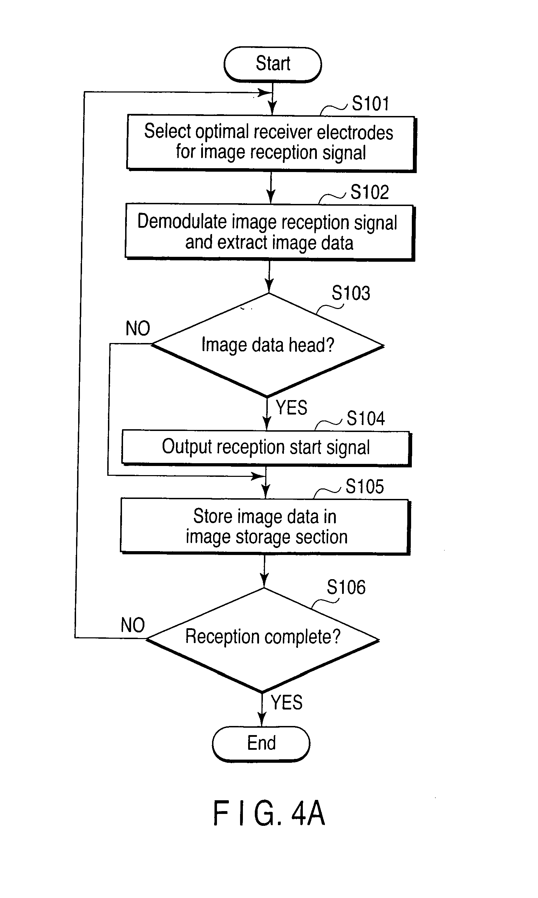

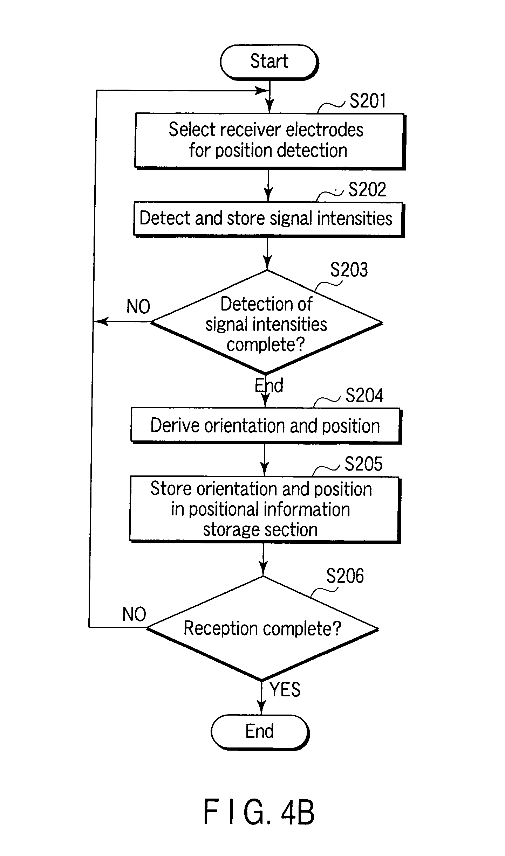

FIG. 4A is a flowchart representing the extraction operation for image data in the processing device 7. FIG. 4B is a flowchart representing the position detection operation in the processing device 7.

The extraction operation for image data in the processing device 7 will be described first with referenced to FIG. 4A. When the power supply circuit 9 of the capsule endoscope 2 is started up, whether the capsule endoscope 2 operates normally or not is then checked. If the capsule endoscope 2 is checked to be operating normally, the capsule endoscope 2 is then introduced into the body of the test subject 1. After the capsule endoscope 2 is introduced into the body of the test subject 1, the system control circuit 17 causes the imaging element drive circuit 13 to drive the CCD 12, thereby to perform imaging of interior of the test subject. Thereafter, the system control circuit 17 obtains image data of interior of the test subject by performing processing, such as gain correction, color temperature correction, and data compression, on image signals obtained by imaging of the interior of the test subject. Image data obtained by the system control circuit 17 is output to the test subject 1 through the modulation circuit 14, matching circuit 15, and transmitter electrodes 16a and 16b. A transmission signal from the transmitter electrodes 16a and 16b reaches the receiver electrodes 6a to 6n provided on a body surface of the test subject 1 through the test subject 1 as a transfer medium.

The signal processing circuit 24 in the processing device 7 outputs a receiver electrode switching signal to the receiver electrode selection circuit 18 so as to select optimal paired receiver electrodes for receiving the transmission signal (image reception signal) from the capsule endoscope 2, from among the receiver electrodes 6a to 6n. Upon receiving this switching signal, the receiver electrode selection circuit 18 selects receiver electrodes (step S101). For the first time, the receiver electrode selection circuit 18 sequentially selects paired receiver electrodes, and uses, as paired receiver electrodes for receiving the transmission signal, such paired receiver electrodes that obtain an optimal reception intensity. For the second time and later, optimal receiver electrodes are selected based on the orientation and position of the capsule endoscope 2 which are derived by the positional information derivation section 25. Such selection of receiver electrodes may be performed each time when a transmission signal is received from the capsule endoscope 2. Alternatively, the selection may be performed only when existing selected receiver electrodes need be switched based on the orientation and position of the capsule endoscope 2, e.g., only when an intensity of a reception signal remarkably drops with use of presently selected paired receiver electrodes.

After receiver electrodes are selected by the receiver electrode selection circuit 18, a transmission signal is received from the capsule endoscope 2 through the selected receiver electrodes. The image reception signal is subjected to processing, such as amplification, by the amplification circuit 19, and is then demodulated by the receiver circuit 20 and input to the signal processing circuit 24. The signal processing circuit 24 extracts image data from the signal demodulated by the receiver circuit 20 (step S102). Thereafter, the signal processing circuit 24 determines whether the extracted image data constitutes a head of image data of interior of a test subject or not (step S103). For example, this determination is performed by detecting data indicating image data as a head, wherein data (for example, a vertical synchronization signal) indicating the image data as a head is configured to be transmitted together with the image data when transmitting a transmission signal from the capsule endoscope 2 for the first time. If the extracted image data is determined in the step S103 to constitute a head of image data of interior of the test subject, the signal processing circuit 24 outputs a reception start signal to the positional information derivation section 25 (step S104). Otherwise, if the extracted image data is not determined in the step S103 to constitute a head of image data of interior of the test subject, the signal processing circuit 24 skips operation of the step S104.

Thereafter, the signal processing circuit 24 causes the image storage section 26 to store the extracted image data (step S105). Data of a reception intensity which is output to the positional information derivation section 25 is stored in the unillustrated memory in the positional information derivation section 25. Thereafter, the signal processing circuit 24 determines whether reception of a transmission signal from the capsule endoscope 2 is complete or not (step S106). If reception of the transmission signal from the capsule endoscope 2 is not determined to be complete in the step S106, the processing returns to the step S101. Otherwise, if reception of the transmission signal from the capsule endoscope 2 is determined to be complete in the step S106, e.g., if no transmission signal is received from the capsule endoscope 2 for a predetermined time period, the processing of FIG. 4A ends.

The processing represented in FIG. 4A relates to extraction of image data of interior of a test subject, which is obtained by the capsule endoscope 2 in the intra-subject position detection system.

Next, position detection operation in the processing device 7 will be described with reference to FIG. 4B. The position detection electrode selection circuit 21 starts operating, triggered by a position detection electrode switching signal which is output from the positional information derivation section 25 upon receiving a reception start signal. In accordance with the position detection electrode switching signal, the position detection electrode selection circuit 21 selects, as paired receiver electrodes for position detection, a set of receiver electrodes other than the receiver electrodes which are selected by the receiver electrode selection circuit 18 and used for receiving an image reception signal (step S201).

After receiver electrodes are selected by the position detection electrode selection circuit 21, a transmission signal from the capsule endoscope 2 is received through the selected receiver electrodes. The signal received is subjected to processing such as amplification by the amplification circuit 22. Thereafter, the detection circuit 23 detects a signal intensity of the reception signal. Data of the signal intensity detected by the detection circuit 23 is stored, together with information of the presently selected receiver electrodes for position detection, in the unillustrated memory in the positional information derivation section 25 through the signal processing circuit 24. Further, the intensity of an image reception signal received by the receiver electrodes selected by the receiver electrode selection circuit 18 is detected by the detection circuit 23, and stored in the memory in the positional information derivation section 25 (step S202).

Thereafter, the positional information derivation section 25 determines whether detection of signal intensities is complete or not, i.e., whether signal intensities have been detected for all combinations of receiver electrodes that are required for orientation and position detection (step S203). If detection of signal intensities is not determined to be complete in the step S203, the processing returns to the step S201. Further, the positional information derivation section 25 selects other receiver electrodes for position detection. Otherwise, if the detection of signal intensities is determined to be complete in the step S203, the positional information derivation section 25 derives the orientation and position of the capsule endoscope 2 from signal intensities of a received signal through respective pairs of paired receiver electrodes selected by the position detection electrode selection circuit 21 and from a signal intensity of an image reception signal through the paired receiver electrodes selected by the receiver electrode selection circuit 18 (step S204). Next, the positional information derivation section 25 stores orientational/positional data of the capsule endoscope 2 in the positional information storage section 27, with the data associated with image data stored in the image storage section 26 (step S205). Further, the orientational/positional data is output to the signal processing circuit 24. Upon necessity, the signal processing circuit 24 selects receiver electrodes for an image reception signal, based on the orientation and position.

Thereafter, the positional information derivation section 25 determines whether reception of a transmission signal from the capsule endoscope 2 is complete or not (step S206). If reception of the transmission signal from the capsule endoscope 2 is not determined to be complete in the step S206, the processing returns to the step S201. Otherwise, if the reception of the transmission signal from the capsule endoscope 2 is determined to be complete in the step S206, the processing of FIG. 4B ends.

The processing represented in FIG. 4B relates to detection of the orientation and position of the capsule endoscope 2 in the intra-subject position detection system.

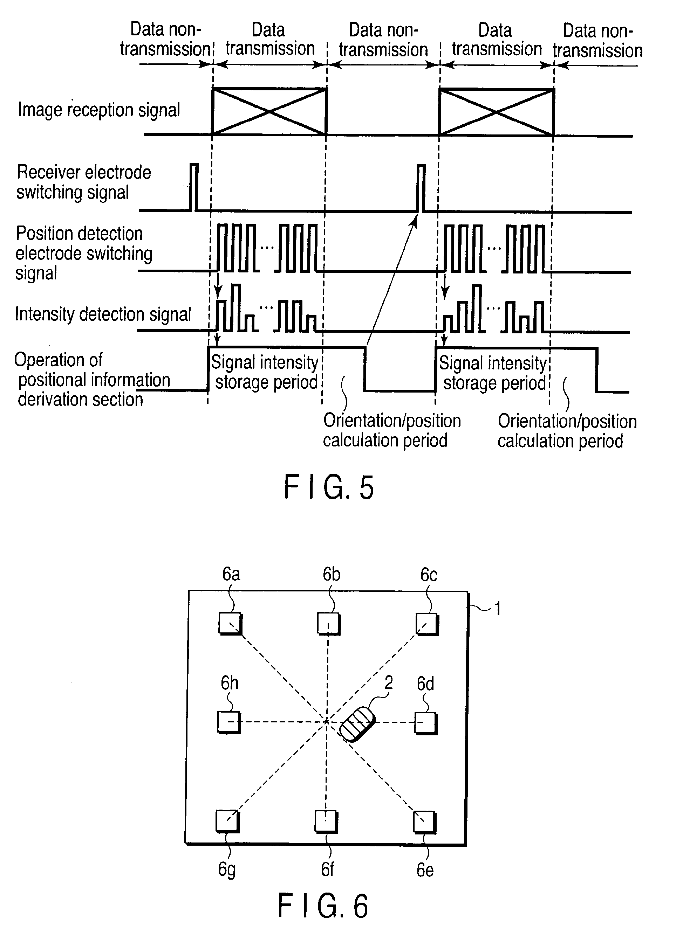

FIG. 5 is a chart representing operation timings of image extraction processing and position detection processing.

As represented in FIG. 5, transmission of the transmission signal from the capsule endoscope 2 is performed in a manner that a data transmission period of transmitting a transmission signal and a data non-transmission period of transmitting no transmission signal are repeated at a constant interval (a frame rate).

When the receiver electrode switching signal from the signal processing circuit 24 is switched from a low level to a high level, receiver electrodes for an image reception signal are then selected. In this manner, the transmission signal from the capsule endoscope 2 is repeatedly received, at a constant interval, as an image reception signal appearing through the selected receiver electrodes. If the signal processing circuit 24 recognizes (for example, depending on a vertical or horizontal synchronization signal inserted at a head of image data) that a data transmission period has been started, the positional information derivation section 25 then transmits a position detection electrode switching signal to the position detection electrode selection circuit 21. A signal intensity of a signal received through the selected receiver electrodes is stored in the unillustrated memory in the positional information derivation section 25, with the signal intensity associated with a combination of selected receiver electrodes at this time. Further, signal intensities are sequentially stored in the memory while switching receiver electrodes for position detection within the data transmission period of the capsule endoscope 2.

When a number of signal intensities enough to detect an orientation and a position are stored in the memory, the orientation and position of the capsule endoscope 2 and an optimal combination of receiver electrodes for image reception are derived from reception intensities through the receiver electrodes for position detection and from reception intensities through the receiver electrodes for image reception. In the example of FIG. 5, derivation of the orientation and position and derivation of optimal receiver electrodes are performed after signal intensities are detected for all combinations of receiver electrodes for position detection. However, the orientation and position and the optimal receiver electrodes may be derived before signal intensities of all such combinations are stored in the memory. Specifically, based on an orientation and a position of the capsule endoscope 2 which have been derived for a previous time, signal intensities may be detected for only a part of all combinations of receiver electrodes for position detection, and a present orientation and a present position of the capsule endoscope 2 may then be derived. In this case, derivation of the orientation and position and derivation of the optimal receiver electrodes may be performed within the data transmission period.

In case of changing receiver electrodes for image reception depending on a result of deriving optimal receiver electrodes for image reception, a receiver electrode switching signal may be transmitted from the signal processing circuit 24 to the receiver electrode selection circuit 18 within the data non-transmission period of the capsule endoscope 2. In this manner, the receiver electrodes are not changed during the data transmission period of the capsule endoscope 2 but the transmission signal from the capsule endoscope 2 can be properly received.

FIG. 6 is a plan view for describing a procedure of selecting receiver electrodes for position detection. FIG. 6 is a schematic view illustrating that eight (n = 8) receiver electrodes 6a to 6h are provided on a body surface of a test subject 1 and the capsule endoscope 2 introduced into the test subject 1 exists near the receiver electrodes 6a to 6h.

In order to derive the orientation and position of the capsule endoscope 2 and optimal receiver electrodes for receiving an image reception signal, receiver electrodes for position detection may be switched within the data transmission period of the capsule endoscope 2, as described above. The orientation and position and the optimal receiver electrodes may then be obtained by calculation from a reception intensity obtained for each time receiver electrodes are switched.

A method for preferentially selecting paired receiver electrodes between which a longer inter-electrode distance exists is considered as a procedure of selecting paired receiver electrodes for detecting the orientation of the capsule endoscope 2 at high speed. Specifically, selection is started in an order from paired receiver electrodes between which a longer inter-electrode distance exists, such as 6a-6e, 6b-6f, 6c-6g, and 6d-6g among the receiver electrodes arranged as illustrated in FIG. 6. If the capsule endoscope 2 exists at the position illustrated in FIG. 6, the combination of 6c-6g most increases the reception intensity on condition that the foregoing four pairs of receiver electrodes are given. Accordingly, the orientation of the capsule endoscope 2 is revealed to be close to the axis 6c-6g. After reception intensities are detected on condition that the foregoing four pairs of receiver electrodes are given, reception intensities are preferably detected for paired reception intensity between which a next longer inter-electrode distance exists. Specifically, pairs of receiver electrodes 6b-6h, 6f-6h, 6d-6f, and 6b-6d are selected among the receiver electrodes arranged as illustrated in FIG. 6, and reception intensities thereof are detected.

Alternatively, a method of sequentially switching one receiver electrode while regarding another receiver electrode as a reference on the basis of a previously derived position is also considered as a procedure of selecting paired receiver electrodes for detecting the orientation of the capsule endoscope 2 at high speed. As an example, if a previously derived position of the capsule endoscope 2 is as illustrated in FIG. 6, selection for another receiver electrode is started while the receiver electrode 6d which is closest to the capsule endoscope 2 is regarded as a reference electrode. In this case, position detection accuracy can be improved corresponding to the number of reception intensities to be obtained, i.e., the number of times the selection for a receiver electrode is performed.

FIG. 6 illustrates an example in which receiver electrodes are provided two-dimensionally. However, the three-dimensional orientation and position of the capsule endoscope 2 can be detected by three-dimensionally arranging the receiver electrodes.

Next, effects of the intra-subject position detection system according to the first embodiment will be described. As described above, the receiver electrode selection circuit 18 and the position detection electrode selection circuit 21 are individually operated within a period in which the position detection device 3 receives a transmission signal from the capsule endoscope 2. In this manner, the signal intensity of each paired receiver electrodes can be detected by switching paired receiver electrodes selected for receiving an image reception signal and paired receiver electrodes selected for position detection. As a result, an extraction function to extract image data and a detection function to detect an orientation and a position can be performed independently from each other within one single period. Therefore, an accurate position at the time when the capsule endoscope 2 picks up an image can be detected at high speed. Further, detection at a much higher speed can be performed by providing procedures of selecting receiver electrodes for position detection respectively for orientation detection and for position detection.

In addition, in the capsule endoscope 2, the transmission signal has a directivity owing to a configuration of transmitter electrodes provided on an outer circumferential surface of the capsule endoscope 2. Therefore, the reception intensity of a signal received by receiver electrodes varies depending on changes in orientation of the capsule endoscope 2 in the test subject 1. According to the present embodiment, the extraction function for image data and the detection function for the orientation and position can be performed independently from each other. Therefore, position detection can be performed immediately in response to a signal intensity of receiver electrodes which has varied. In this manner, a time which is required selecting receiver electrodes again is shortened. Therefore, an effect of being capable of stably receiving a reception signal can be achieved.

### [Second Embodiment]

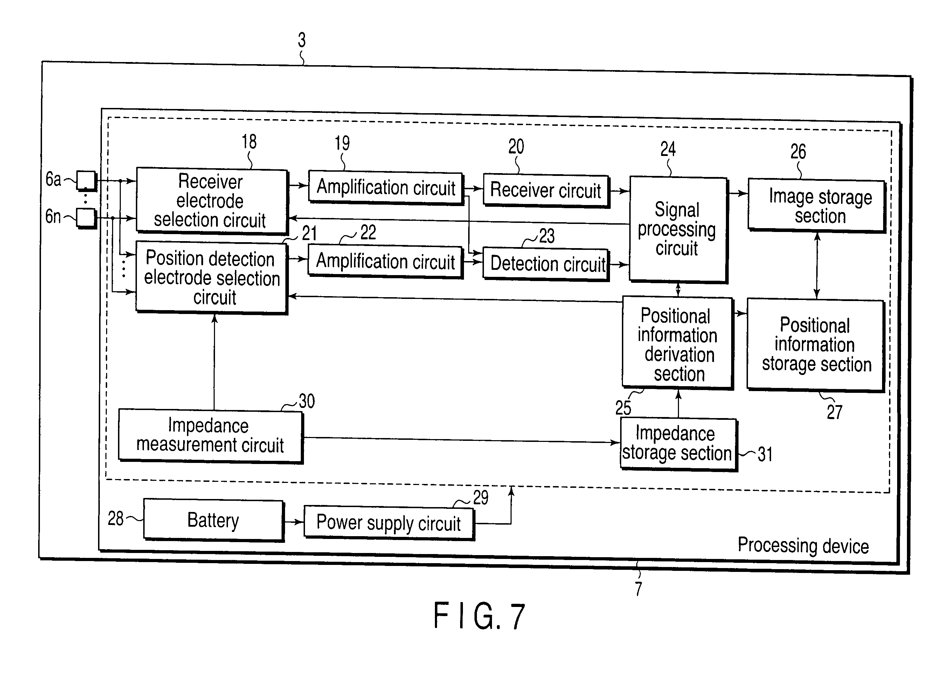

Next, the second embodiment of the invention will be described. FIG. 7 is a block diagram representing a detailed interior configuration of a position detection device 3 according to an intra-subject position detection system according to the second embodiment of the invention. Parts of the configuration of FIG. 7 which are common to the configuration of FIG. 3 are denoted at common reference symbols, and descriptions thereof will be omitted herefrom. In addition, the capsule endoscope 2 has the same configuration as that in the first embodiment, and a description thereof will be therefore omitted as well.

In addition to the interior configuration of the processing device 7 represented in FIG. 3, a processing device 7 represented in FIG. 7 includes an impedance measurement circuit 30 which detects an impedance between paired receiver electrodes selected by a position detection electrode selection circuit 21 from among receiver electrodes 6a to 6n, and an impedance storage section 31 which stores impedances measured by the impedance measurement circuit 30. An auto-balance bridge method using an I-V converter can be used as a measurement method in which the impedance measurement circuit 30 measures an impedance. A further alternative will be a branch line coupler or an orientational coupling circuit which transmits an impedance measurement signal to between receiver electrodes and performs impedance detection by using a component of a reflected signal thereof.

The timing at which impedances are firstly measured between receiver electrodes is before the capsule endoscope 2 is introduced into a test subject 1. Further, normality of operation of the capsule endoscope 2 is checked, and the capsule endoscope 2 is then introduced into the test subject 1. Thereafter, impedances are measured in each data non-transmission period of the capsule endoscope 2.

As described above, the positional information derivation section 25 derives the orientation and position of the capsule endoscope 2 from a signal intensity of paired receiver electrodes for position detection. However, alternatively according to the second embodiment, a current intensity may be calculated not only from a voltage intensity of a signal received but also from a impedance between the receiver electrodes which is stored in the impedance storage section 31. The orientation and position of the capsule endoscope 2 may then be obtained from the current intensity. Further, the positional information derivation section 25 has a function to transmit a control signal to the signal processing circuit 24, depending on an impedance stored in the impedance storage section 31. Specifically, if an impedance between receiver electrodes is high, the positional information derivation section 25 transmits an alarm control signal to the signal processing circuit 24. In response to the alarm control signal, the signal processing circuit 24 detects a non-contact state between the receiver electrodes and the test subject 1.

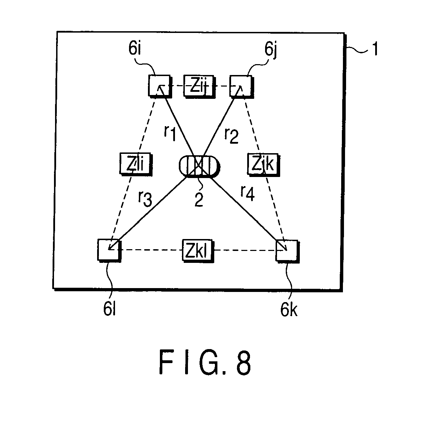

FIG. 8 is a schematic view for describing position detection of the capsule endoscope 2 by the position detection device 3 according to the second embodiment, using together an impedance between receiver electrodes. If the capsule endoscope 2 exists at the position illustrated in FIG. 8, a signal intensity of paired receiver electrodes 6a-6j and a signal intensity of paired receiver electrodes 61-6k are found to be influenced by an inter-electrode distance between the receiver electrodes each other and by a distance between the receiver electrodes and the capsule endoscope 2 from a comparison between both signal intensities.

In this respect, influence from an error by an inter-electrode distance can be reduced by measuring an inter-electrode impedance and by using the inter-electrode impedance for position detection. For example, if a distance between receiver electrodes and an impedance between the receiver electrodes differs from each other (for example, Zij > Zkl : the inter-electrode distance is small while the inter-electrode impedance is large), accuracy of position detection is higher when detecting the orientation and position by use of a current intensity depending on an impedance than when detecting the position by a signal intensity.

Where paired receiver electrodes 6i-6j and 6j-6k are compared with each other, the capsule endoscope 2 is assumed to exist at a position where the paired receiver electrodes 6i-6j and the paired receiver electrodes 6j-6k are line-symmetrical to each other insofar as impedances Zij and Zjk respectively between the receiver electrodes 6i-6j and between the receiver electrodes 6j-6k are equal to each other. However, if impedances between receiver electrodes vary in accordance with elapse of time, a level difference occurs between both signal intensities and may easily cause an error in position detection based on signal intensities. In this respect, the second embodiment periodically measures impedances between receiver electrodes and uses the impedances for position detection. Therefore, position detection can be performed with higher accuracy than position detection using only signal intensities.

FIG. 8 illustrates an example in which receiver electrodes are arranged two-dimensionally. However, a three-dimensional orientation of the capsule endoscope 2 can be detected by three-dimensionally arranging the receiver electrodes.

Next, effects of the intra-subject position detection system according to the second embodiment will be described. In the configuration as described above, current intensities which can be calculated from voltage intensities and impedances between receiver electrodes provided on a body surface of the test subject 1 are used for current detection. Therefore, the orientation and position of the capsule endoscope 2 can be detected at higher accuracy.

Further, impedances between receiver electrodes are periodically detected. Therefore, even when a contact state between the receiver electrodes and the test subject 1 changes, highly accurate position detection can be maintained.

The present invention has been described above on the basis of embodiments. The invention, however, is not limited to the embodiments described above but can of course be variously modified and applied within the scope of the subject matter of the invention.

Further, the above embodiments cover various phases of the invention. Various inventions can further be derived from appropriate combinations of disclosed plural componential elements. For example, if problems as described above can be solved and effects as also described above can be obtained with several componential elements omitted from the whole componential elements suggested in the above embodiments, the configuration omitting the several components can be derived as invention.

## Claims

1. An intra-subject position detection system comprising:
an intra-subject introduction device that is introduced into a test subject and obtains an intra-subject information; and
a position detection device that is provided outside the test subject and derives a positional information of the intra-subject introduction device inside the test subject, **characterized in that**
the intra-subject introduction device comprises transmitter electrodes that are positioned on an outer circumferential surface of the intra-subject introduction device, to transmit the intra-subject information to outside of the test subject, and
the position detection device comprises
a plurality of receiver electrodes provided on an outer surface of the test subject,
a receiver electrode selection circuit that selects receiver electrodes for receiving the intra-subject information, from among the plurality of receiver electrodes,
a position detection electrode selection circuit that selects receiver electrodes for position detection of the intra-subject introduction device, from among the other receiver electrodes of the plurality of receiver electrodes than the receiver electrodes selected by the receiver electrode selection circuit, and
a positional information derivation circuit that derives the positional information of the intra-subject introduction device, based on an electric signal from the receiver electrodes selected by the position detection electrode selection circuit.

2. The intra-subject position detection system according to claim 1, **characterized in that** the positional information derivation circuit detects the electric signal while receiving the intra-subject information, and derives the positional information of the intra-subject introduction device before a next intra-subject information is received.

3. The intra-subject position detection system according to claim 1, **characterized in that**
the position detection device further comprises an impedance measurement circuit that measures impedance between the plurality of receiver electrodes, and
the positional information derivation circuit derives the positional information of the intra-subject introduction device, based on an electric signal from the receiver electrode selected by the position detection electrode selection circuit and based on the impedance measured by the impedance measurement circuit.

4. The intra-subject position detection system according to claim 1, **characterized in that** the receiver electrode selection circuit selects receiver electrodes for receiving a next intra-subject information, based on the positional information of the test subject introduction device derived by the positional information derivation circuit.

5. The intra-subject position detection system according to claim 4, **characterized in that** the receiver electrode selection circuit selects the receiver electrodes for receiving the next intra-subject information while the intra-subject information is not being received.

6. The intra-subject position detection system according to claim 1, **characterized in that** the position detection device further comprises a band-pass filter that allows a signal within a band to pass, among position detection signals received by the receiver electrodes, the band being centered on a frequency of the position detection signals.

7. The intra-subject position detection system according to claim 1, **characterized in that** the position detection device smoothens a position detection signal received by the receiver electrodes, and thereafter detects and outputs an amplitude of the position detection signal to the positional information derivation circuit.

8. The intra-subject position detection system according to claim 1, **characterized in that** the position detection device detects an effective value of a position detection signal received by the receiver electrodes, and outputs the effective value to the positional information derivation circuit.

9. The intra-subject position detection system according to claim 1, **characterized by** comprising a storage section that stores the intra-subject information and positional information of the intra-subject introduction device which are obtained at one identical timing, with the intra-subject information and the positional information associated with each other.

10. The intra-subject position detection system according to claim 1, **characterized in that** the position detection electrode selection circuit selects a pair of receiver electrodes used for position detection of the intra-subject introduction device in an order of length of inter-electrode distance.

11. The intra-subject position detection system according to claim 4, **characterized in that** the position detection electrode selection circuit selects one receiver electrode for receiving the next intra-subject information based on the positional information of the intra-subject introduction device which has been detected for a previous time, and selects another receiver electrode by sequentially switching the receiver electrodes while regarding the one receiver electrode as a reference.

12. The intra-subject position detection system according to claim 1, **characterized in that** the receiver electrodes are three-dimensionally arranged on the outer surface of the test subject.

13. An intra-subject position detection method for deriving a positional information of an intra-subject introduction device that is introduced into a test subject and obtains intra-subject information, the method **characterized by** comprising:
selecting receiver electrodes for receiving the intra-subject information, from among a plurality of receiver electrodes provided on an outer surface of the test subject;
selecting, from among the other receiver electrodes of the plurality of receiver electrodes than the selected receiver electrodes, receiver electrodes for a position detection of the intra-subject introduction device; and
deriving the positional information of the intra-subject introduction device, based on an electric signal transmitted from transmitter electrodes and received by the receiver electrodes for the position detection, the transmitter electrodes being provided on an outer circumferential surface of the intra-subject introduction device, to transmit the intra-subject information to outside of the test subject.
